# EUROPEAN PATENT SPECIFICATION

(11) **EP 4 146 225 B1**
(45) Date of publication and mention of the grant of the patent: **09.10.2024**
(21) Application number: 21724580.2
(22) Date of filing: 30.04.2021
(51) Int. Cl.: A61K 31/5415, A61K 33/00, A61P 7/00, A61P 11/00, A61P 43/00

(54) **METHYLTHIONINIUM COMPOUNDS FOR USE IN THE TREATMENT OF HYPOXEMIA**
METHYLTHIONINIUM-VERBINDUNGEN ZUR VERWENDUNG BEI DER BEHANDLUNG VON HYPOXÄMIE
COMPOSÉS MÉTHYLTHIONINIUM DESTINÉS À ÊTRE UTILISÉS DANS LE TRAITEMENT DE L'HYPOXÉMIE

(30) Priority: 05.05.2020 GB 202006659; 26.10.2020 GB 202016957
(43) Date of publication of application: 15.03.2023
(73) Proprietor: WisTa Laboratories Ltd., Singapore 658066 (SG)
(72) Inventor: ARASTOO, Mohammad, Aberdeen Aberdeenshire AB25 2ZE (GB); MAZANETZ, Michael Philip, Glasgow G43 2NX (GB); WISCHIK, Claude Michel, Aberdeen Aberdeenshire AB24 5RP (GB)
(74) Representative: Mewburn Ellis LLP
(86) International application number: PCT/EP2021/061485
(87) International publication number: WO 2021/224146

(56) References cited:
- WO-A1-02/04025
- WO-A1-2012/107706
- WO-A1-2020/020751
- WO-A1-2020/250186
- DARYOUSH HAMIDI ALAMDARI ET AL: "The Application of a Reduced Dye Used in Orthopedics as a Novel Treatment against Coronavirus (COVID-19): A Suggested Therapeutic Protocol", THE ARCHIVES OF BONE AND JOINT SURGERY, 1 April 2020 (2020-04-01), Mashhad, Iran, pages 291 - 294, XP055762802, Retrieved from the Internet <URL:https://www.ncbi.nlm.nih.gov/pmc/articles/PMC7296601/pdf/ABJS-8-291.pdf> [retrieved on 20210629], DOI: 10.22038/abjs.2020.47745.2349
- T. C. BADDELEY ET AL: "Complex Disposition of Methylthioninium Redox Forms Determines Efficacy in Tau Aggregation Inhibitor Therapy for Alzheimer's Disease", JOURNAL OF PHARMACOLOGY AND EXPERIMENTAL THERAPEUTICS, vol. 352, no. 1, 15 October 2014 (2014-10-15), pages 110 - 118, XP055384953, DOI: 10.1124/jpet.114.219352
- BRADBERRY SALLY ED - HARRISON CLAIRE ET AL: "Methaemoglobinaemia", MEDICINE - U K EDITION, THE MEDICINE PUBLISHING COMPANY, GB, vol. 44, no. 2, 6 February 2016 (2016-02-06), pages 91 - 92, XP029439264, ISSN: 1357-3039, DOI: 10.1016/J.MPMED.2015.11.018
- MIYAMOTO AKIKO ET AL: "Effect of chronic methylene blue administration on hypoxemia in rats with common bile duct ligation : Methylene blue in rats with hypoxemia", HEPATOLOGY RESEARCH, vol. 40, no. 6, 14 April 2010 (2010-04-14), NL, pages 622 - 632, XP055820697, ISSN: 1386-6346, DOI: 10.1111/j.1872-034X.2010.00640.x

## Description

### Technical field

The present invention relates generally to materials for use for alleviating hypoxemia or treatment of hypoxia in a subject.

### Background art

One of the primary functions of the cardiorespiratory system, including the blood, is to ensure that all tissues are adequately oxygenated at all times, i.e., that the pO₂ in the immediate environment of a cell exceeds the critical pO₂ needed for normal mitochondrial oxygen consumption and ATP production (see Chapter 7, Pittman RN. Regulation of Tissue Oxygenation. San Rafael (CA): Morgan & Claypool Life Sciences; 2011)

It is the role of various regulatory mechanisms in the cardiovascular system, respiratory system and blood to ensure proper oxygenation of the tissues. Deviations from normal values of the key variables of oxygen transport, from many different causes, can lead to hypoxic tissue environments and resulting tissue damage or morbidity

Thus it can be seen that providing compounds which can be used safely to enhance the oxygen carrying capacity (saturation) of the blood provides a useful contribution to the art.

### Disclosure of the invention

Note that the references to methods of treatment in the description of the invention are to be interpreted as references to the compounds, pharmaceutical compositions and medicaments of the present invention for use in a method for treatment of the human (or animal) body by therapy.

The present invention provides for the use of certain hydromethylthionine salts (referred to as "LMTX" below) as therapeutics for alleviating hypoxemia in subjects. This may in turn be used to alleviate hypoxia and treat pathologies or other causes of hypoxia.

MTC (methylthionium chloride, methylene blue) is an FDA and EMA approved drug with a long history of clinical use.

LMTX delivers the same MT (methylthionine) moiety systemically, but is more suitable for oral and intravenous use than MTC as it has improved absorption, red cell penetration and deep compartment distribution (Baddeley et al., 2015). LMTX can be used at a substantially lower dose than MTC and is thus better tolerated.

It was recently reported (Alamdari, Daryoush Hamidi, et al. "Application of methylene blue-vitamin C-N-acetyl cysteine for treatment of critically ill COVID-19 patients, report of a phase-I clinical trial." European Journal of Pharmacology 885 (2020): 173494 that methylene blue-vitamin C-N-acetyl Cysteine (MCN) provided benefits to critically ill COVID-19 patients, with one presumptive mechanism of this agent and dosage being via reduction in methaemoglobin (metHb) (see Conclusions therein).

Akiko Miyamoto et al.; Hepatology Research, 2010, 40: 622-632; discloses the effect of chronic methylene blue administration on hypoxemia in rats with common bile duct ligation.

Based on *in vivo* evidence from controlled clinical trials, the present inventors demonstrate that LMTX salts can enhance oxygen saturation even at relatively low doses, and unrelated to any known effects on metHb.

Without wishing to be bound by theory, the inventors propose that the binding of the LMT moiety to haemoglobin overcomes the initial energy barrier for oxygen binding, which thereby facilitates subsequence binding and oxygenation of all four heme groups of haemoglobin.

WO2007/110627 disclosed certain 3,7-diamino-10H-phenothiazinium salts, effective as drugs or pro-drugs for the treatment of diseases including Alzheimer's disease and other diseases such as Frontotemporal dementia (FTD), as well as viral diseases generally. These compounds are also in the "reduced" or "leuco" form when considered in respect of MTC. These leucomethylthioninium compounds were referred to therein as "LMTX" salts. WO2012/107706 described other LMTX salts having superior properties to the LMTX salts listed above, including leuco-methylthioninium bis(hydromethanesulfonate) (LMTM) (WHO INN designation: hydromethylthionine):

| | |
|---|---|
| 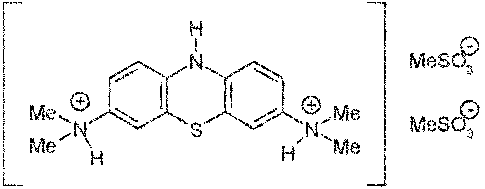 | *N,N,N',N*'-tetramethyl-10*H-*phenothiazine-3,7-diaminium bis(hydromethanesulfonate). |
| | LMT.2MsOH / LMTM |

LMTX have not previously been disclosed for the treatment of hypoxemia.

Thus in one aspect there is disclosed an MT-containing compound for use in a method of treating (or alleviating) hypoxemia in a subject,
which method comprises orally administering to said subject a methylthioninium (MT)-containing compound,
wherein said administration provides a total daily oral dose of 0.5 mg to 250 mg of MT to the subject per day, optionally split into 2 or more doses,
wherein the MT-containing compound is an LMTX compound of the following formula:

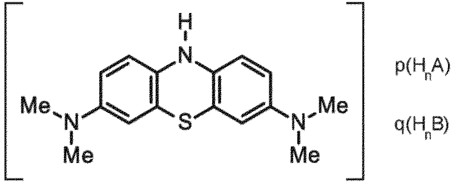

wherein each of HₙA and HₙB (where present) are protic acids which may be the same or different,
and wherein p = 1 or 2; q = 0 or 1; n = 1 or 2; (p + q) × n = 2,
or a hydrate or solvate thereof.

In some embodiments said administration provides a total daily oral dose of more than 0.5, 5, 10, 15, 20, 25, 30, 35, 40, 50, or 60 mg and less than or equal to 100, 150, 200 or 250 mg of MT to the subject per day, optionally split into 2 or more doses.

In one embodiment said administration provides a total daily oral dose of more than 35, 40, 50, or 60 mg and less than or equal to 100, 150, 200 or 250 mg of MT to the subject per day, optionally split into 2 or more doses.

The total daily oral dose may be greater than or equal to 30.5, 30.6, 31, 35, 37.5, 40, 45, 50, 55, 60, 65, 70, 75, 80, 85, 90, 95, 100, 105, 110, 115, 120, 125, 130, 140, 150, 160, 170, 180, 200, 210, 220, 230, 240, or 250 mg.

The total daily oral dose may be 60, 75, or 120 mg.

***

In some embodiments it may be preferred to use a relatively low dose, in order to minimise any risk of causing Met-Hb when alleviating hypoxemia. As explained in the Examples hereinafter, even doses as low as 4mg of MT provided as LMTX have shown clinical benefit.

Thus the total dose may be from around any of 0.5, 1, 1.5, 2, 2.5, 3, 3.5, 4 mg to around any of 5, 6, 7, 8, 9, 10, 11, 12, 13, 14, 15, 16, 17, 18, 19, or 20mg.

An example dosage is 1 to 20mg.

An example total daily dose is about 0.5, 1, 1.5, 2, 2.5, 3, 3.5, 4, 5, 6, 7, 8, 9, 10, 11, 12, 13, 14, 15, 16, 17, 18, 19, or 20mg.

A further example dosage is 2 to 15 mg.

The total dose may be from around any of 0.5, 1, 1.5, 2, 2.5, 3, 3.5, 4 mg to around any of 5, 6, 7, 8, 9 or 10mg.

A further example dosage is 3 to 10 mg.

A further preferred dosage is 3.5 to 7 mg.

A further preferred dosage is 4 to 6 mg.

The total daily dose of the compound may be administered as a split dose twice a day or three times a day.

As explained below, when administering the MT dose split in a larger number of doses/day it may be desired to use a smaller total amount within the recited range, compared to a single daily dosing, or a smaller number of doses per day.

***

The subject for treatment may be characterised or selected by certain criteria.

For the present invention the subject must be able to breathe and swallow if treatment is to be administered orally.

Blood oxygen saturation levels (SpO₂) of around 94% or 95% or greater are generally considered to be normal. SpO₂ <94% suggests hypoxaemia. Breathless patients with a saturation of ≤92% at room air are likely to be in respiratory failure. For patients with mild respiratory diseases, the SpO₂ should be 90% or above and preferably 95%.

Therefore the subject is characterised by having a SpO₂ less than 95% on room air e.g. less than or equal to 94%, 93%, 92%, 91% or 90%.

The invention comprises the step of selecting the subject according to one or more of these criteria e.g. having an SpO₂ value as described above. Thus the method of the invention may comprise determining SpO₂, for example by pulse oximetry. Thus in some embodiments the subject may be a human who has been diagnosed as having ("confirmed") hypoxemia, or wherein said method comprises making said diagnosis.

The patient may be an adult human, and the population-based dosages described herein are premised on that basis (typical weight 50 to 70 kg). If desired, corresponding dosages may be utilised for subjects falling outside of this range by using a subject weight factor whereby the subject weight is divided by 60 kg to provide the multiplicative factor for that individual subject.

As noted above, SpO₂ can be conveniently measured using pulse oximetry.

The principle behind pulse oximetry lies in the red and infrared light absorption characteristics of oxygenated and deoxygenated haemoglobin. Oxygenated blood absorbs infrared light more and allows red light to pass through whereas deoxygenated haemoglobin absorbs more red light and allows more infrared light to pass through.

A pulse oximeter has a transmitter that transmits red and infrared light through the body part (usually finger, toe or earlobe) and a photo detector that detects the percentage of oxygenated versus deoxygenated haemoglobin through which the light passes.

The device measures the changing absorbance at each of the wavelengths, allowing it to determine the absorbance due to the pulsating arterial blood alone, excluding the venous blood. The percentage of oxygen saturation calculated is referred to as the percentage SpO₂.

The main indication of pulse oximetry is in the assessment of breathless patients, as it provides valuable information about the severity of the illness.

The present invention concerns methods of treating (alleviating) hypoxaemia in a subject i.e. low levels of oxygen in blood. The methods are intended to enhance oxygen carrying capacity of the blood, and increase oxygen saturation in the blood. In some embodiments the methods increase oxygen saturation within 4 hours of administration. As disclosed herein, LMTM is able to increase oxygen saturation in the blood, apparently by a novel mechanism unrelated to its known effects on metHb.

This may in turn be used to treat conditions causing or resulting from hypoxia (inadequate oxygen available for use by the tissues) or anoxia (absence of oxygen being delivered to the tissue).

Since LMTX is demonstrated to directly ameliorate hypoxemia, the precise pathological or environmental cause of that does not limit the scope of the invention.

For example the hypoxaemia may be anemic hypoxaemia, in which the oxygen carrying capacity of the blood has been reduced. Alternatively is may be hypoxic hypoxaemia or stagnant hypoxaemia (see Pittman RN. Regulation of Tissue Oxygenation. San Rafael (CA): Morgan & Claypool Life Sciences; 2011).

Thus hypoxaemia or hypoxia may result from other causes than anemia e.g. pulmonary, cardiovascular or environmental causes (e.g., pneumonia, high altitude, chronic lung disease, increased shunt from congenital heart disease etc).

The methods described herein may be used to treat diseases resulting in, or arising from, hypoxaemia, and in particular to treat hypoxaemia in these diseases.

The methods described herein may be used to treat a subject diagnosed with diseases resulting in, or arising from, hypoxemia, and in particular to treat hypoxemia in these subjects.

The methods described herein may be used to treat hypoxemic subjects who are selected according to diagnosis of diseases resulting in, or arising from, hypoxemia, in order to increase SpO₂.

The methods described herein may be used to treat diseases requiring long-term oxygen therapy. Examples include Chronic obstructive pulmonary disease, Pulmonary fibrosis Heart failure, Severe long-term asthma, Pulmonary hypertension and Cystic fibrosis.

The methods described herein may be used to treat acute disease, chronic underlying lung disease, or diseases in which tissue delivery of oxygen is impaired e.g. cardiovascular diseases, and in particular to treat hypoxemia in these diseases. Examples are shown below:

| | |
|---|---|
| 1. Acute disease | Lung injury - caused by trauma or infection, which may be bacterial (e.g. tuberculosis), viral (influenza) or fungal |
| | Decreased ventilation due to non-lung injury, e.g. head injury |
| | |
| 2. Chronic, underlying lung disease | Emphysema |
| | Chronic obstructive pulmonary disease |
| | Asbestosis |
| | Interstitial lung disease (including idiopathic pulmonary fibrosis) |
| | Cystic fibrosis |
| | Asthma |
| | |
| 3. Tissue delivery | Congestive cardiac failure |
| | Pulmonary oedema |
| | Cerebrovascular accident (localised failure of O₂ delivery in the brain) |
| | Pulmonary hypertension |
| | |

In some embodiments the methods of the invention are used to treat any one or more of the following diseases in which hypoxemia is present: anaemia (including iron deficiency); ARDS (Acute respiratory distress syndrome); asbestosis; asthma; bronchitis; carbon monoxide poisoning; cerebral hypoxia; cerebral hypoxia induced by excessive G forces (G-LOC); congenital heart defects in children; congenital heart disease in adults; congestive cardiac failure; COPD (chronic obstructive pulmonary disease) exacerbation -worsening of symptoms; COVID-19; cyanide poisoning; cystic fibrosis; deep sea diving; emphysema; histotoxic hypoxia; hypoventilation training; insomnia; intermittent angioedema; interstitial lung disease; intrauterine hypoxia; ischaemic hypoxia; lung injury, caused by trauma or infection, which may be bacterial, viral or fungal; medications, such as certain narcotics and anaesthetics, that depress breathing; pneumonia; pneumothorax (collapsed lung); pulmonary oedema (excess fluid in the lungs); pulmonary embolism (blood clot in an artery in the lung); pulmonary fibrosis (scarred and damaged lungs); pulmonary hypertension; respiratory alkalosis; sleep apnoea; transient ischaemic attack; tuberculosis; tumour hypoxia.

Chronic obstructive pulmonary disease (COPD) is a chronic inflammatory lung disease that causes obstructed airflow from the lungs (see e.g. Halbert, R. J., et al. "Global burden of COPD: systematic review and meta-analysis." European Respiratory Journal 28.3 (2006): 523-532).

Symptoms include breathing difficulty, cough, mucus (sputum) production and wheezing. It's typically caused by long-term exposure to irritating gases or particulate matter, most often from cigarette smoke. People with COPD are at increased risk of developing heart disease, lung cancer and a variety of other conditions.

Emphysema and chronic bronchitis are the two most common conditions that contribute to COPD. These two conditions usually occur together and can vary in severity among individuals with COPD.

Chronic bronchitis is inflammation of the lining of the bronchial tubes, which carry air to and from the air sacs (alveoli) of the lungs. It's characterized by daily cough and mucus (sputum) production.

Emphysema is a condition in which the alveoli at the end of the smallest air passages (bronchioles) of the lungs are destroyed as a result of damaging exposure to cigarette smoke and other irritating gases and particulate matter.

In one embodiment the subject is a human who has been diagnosed as having COVID-19. The method may comprise making said diagnosis.

Diagnosis of COVID-19 may be via any method known in the art. Examples include laboratory testing for the presence of the SARS-CoV-2 virus - for example directly based on the presence of virus itself (e.g. using RT-PCR and isothermal nucleic acid amplification, or the presence of antigenic proteins) or indirectly via antibodies produced in response to infection. Other methods of diagnosis include chest X-ray, optionally in combination with characteristic symptoms as described below (see e.g. Li, Xiaowei, et al. "Molecular immune pathogenesis and diagnosis of COVID-19." Journal of Pharmaceutical Analysis (2020); Fang, Yicheng, et al. "Sensitivity of chest CT for COVID-19: comparison to RT-PCR." Radiology (2020): 200432; Chan, Jasper Fuk-Woo, et al. "Improved Molecular Diagnosis of COVID-19 by the Novel, Highly Sensitive and Specific COVID-19-RdRp/Hel Real-Time Reverse Transcription-PCR Assay Validated In Vitro and with Clinical Specimens." Journal of Clinical Microbiology 58.5 (2020); Tang, Yi-Wei, et al. "The laboratory diagnosis of COVID-19 infection: current issues and challenges." Journal of Clinical Microbiology (2020).

In some embodiments, the hypoxaemia may be in a subject who does not suffer alpha1-antitrypsin deficiency (which may lead to emphysema or cirrhosis.

In some embodiments, the hypoxemia may be in a subject who does not suffer from COVID-19, or, alternatively, in such subjects the dosage of MT may be at least 30 or 31 mg day oral.

Preferably the LMT compound is an "LMTX" compound of the type described in WO2007/110627 or WO2012/107706.

Thus the compound may be selected from compounds of the following formula, or hydrates or solvates thereof:

| | |
|---|---|
| 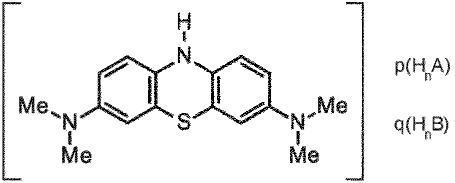 | Options: |
| | p = 1, 2 |
| | q = 0, 1 |
| | n = 1, 2 |
| | (p + q) × n = 2 |

Each of HₙA and HₙB (where present) are protic acids which may be the same or different.

By "protic acid" is meant a proton (H⁺) donor in aqueous solution. Within the protic acid A- or B- is therefore a conjugate base. Protic acids therefore have a pH of less than 7 in water (that is the concentration of hydronium ions is greater than 10⁻⁷ moles per litre).

In one embodiment the salt is a mixed salt that has the following formula, where HA and HB are different mono-protic acids:

| | |
|---|---|
| 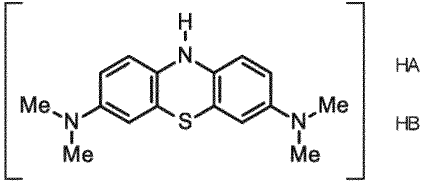 | when: |
| | p = 1 |
| | q = 1 |
| | n = 1 |
| | (1 + 1) × 1 = 2 |

However preferably the salt is not a mixed salt, and has the following formula:

| | |
|---|---|
| 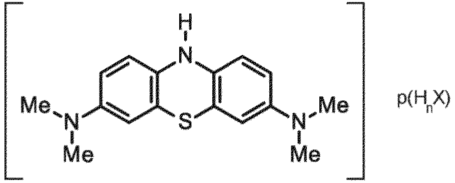 | when: |
| | p= 1, 2 |
| | n = 1, 2 |
| | p × n = 2 |

wherein each of HₙX is a protic acid, such as a di-protic acid or mono-protic acid.

In one embodiment the salt has the following formula, where H₂A is a di-protic acid:

| | |
|---|---|
| 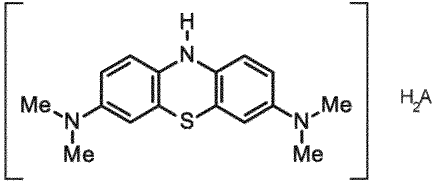 | when: |
| | p = 1 |
| | q = 0 |
| | n = 2 |
| | (1 + 0) × 2 = 2 |

Preferably the salt has the following formula which is a bis monoprotic acid:

| | |
|---|---|
| 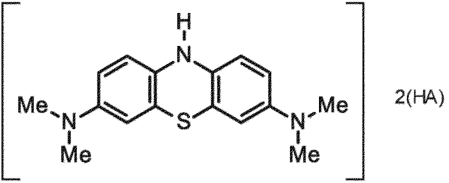 | when: |
| | p = 2 |
| | q = 0 |
| | n = 1 |
| | (2 + 0) × 1 = 2 |

Examples of protic acids which may be present in the LMTX compounds used herein include:
Inorganic acids: hydrohalide acids (e.g., HCl, HBr), nitric acid (HNO₃), sulphuric acid (H₂SO₄)

Organic acids: carbonic acid (H₂CO₃), acetic acid (CH₃COOH), methanesulfonic acid, 1,2-ethanedisulfonic acid, ethansulfonic acid, naphthalenedisulfonic acid, p-toluenesulfonic acid,

Preferred acids are monoprotic acid, and the salt is a bis(monoprotic acid) salt.

A preferred MT compound is LMTM:

| | | | |
|---|---|---|---|
| 1 | 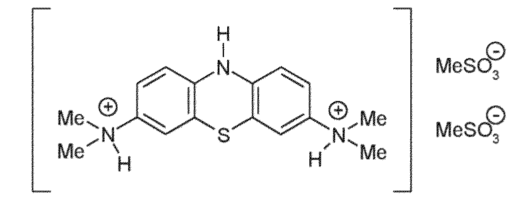 | LMT.2MsOH (LMTM) | 477.6 |
| | | | (1.67) |

### Weight factors

The anhydrous salt has a molecular weight of around 477.6. Based on a molecular weight of 285.1 for the LMT core, the weight factor for using this MT compound in the invention is 1.67. By "weight factor" is meant the relative weight of the pure MT-containing compound vs. the weight of MT which it contains.

Other weight factors can be calculated for example MT compounds herein, and the corresponding dosage ranges can be calculated therefrom.

Other example LMTX compounds are as follows. Their molecular weight (anhydrous) and weight factor is also shown:

| | | | |
|---|---|---|---|
| 2 | 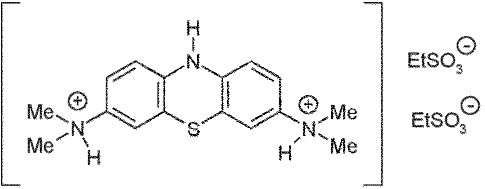 | LMT.2EsOH | 505.7 |
| | | | (1.77) |
| 3 | 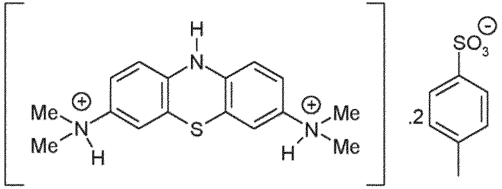 | LMT.2TsOH | 629.9 |
| | | | (2.20) |
| 4 | 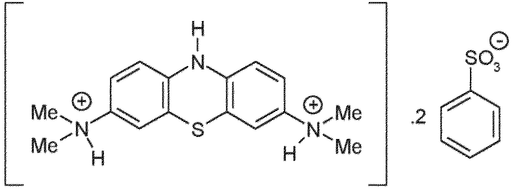 | LMT.2BSA | 601.8 |
| | | | (2.11) |
| 5 | 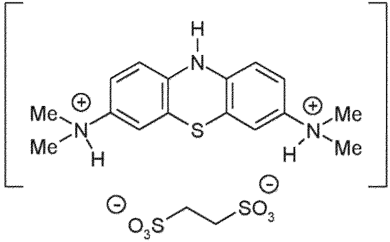 | LMT.EDSA | 475.6 (1.66) |
| 6 | 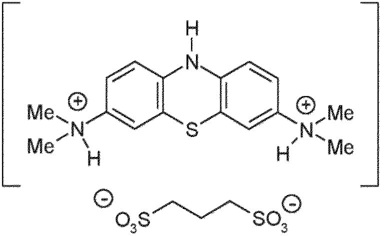 | LMT.PDSA | 489.6 (1.72) |
| 7 | 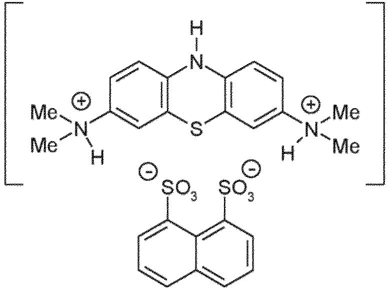 | LMT.NDSA | 573.7 (2.01) |
| 8 | 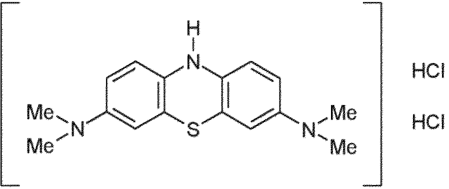 | LMT.2HCl | 358.33 (1.25) |

The dosages described herein with respect to *MT* thus apply *mutatis mutandis* for these MT-containing *compounds,* as adjusted for their molecular weight.

### Accumulation factors

As will be appreciated by those skilled in the art, for a given daily dosage, more frequent dosing can lead to greater accumulation of a drug.

Therefore in certain embodiments of the claimed invention, the total daily dosed amount of MT compound may be relatively lower, when dosing more frequently (e.g. twice a day [bid] or three times a day [tid]), or higher when dosing once a day [qd].

### Treatment and prophylaxis

The term "treatment," as used herein in the context of treating a condition, pertains generally to treatment and therapy, whether of a human or an animal (e.g., in veterinary applications), in which some desired therapeutic effect is achieved, for example, the inhibition of the progress of the condition, and includes a reduction in the rate of progress, a halt in the rate of progress, regression of the condition, amelioration of the condition, and cure of the condition.

The term "therapeutically-effective amount," as used herein, pertains to that amount of a compound of the invention, or a material, composition or dosage from comprising said compound, which is effective for producing some desired therapeutic effect, commensurate with a reasonable benefit/risk ratio, when administered in accordance with a desired treatment regimen. The present inventors have demonstrated that a therapeutically-effective amount of an MT compound in respect of the diseases of the invention can be much lower than was hitherto understood in the art.

The invention also embraces treatment as a prophylactic measure.

The term "prophylactically effective amount," as used herein, pertains to that amount of a compound of the invention, or a material, composition or dosage from comprising said compound, which is effective for producing some desired prophylactic effect, commensurate with a reasonable benefit/risk ratio, when administered in accordance with a desired treatment regimen.

"Prophylaxis" in the context of the present specification should not be understood to circumscribe complete success i.e. complete protection or complete prevention. Rather prophylaxis in the present context refers to a measure which is administered in advance of a condition, or prior to the worsening of such a condition, with the aim of preserving health by helping to delay, mitigate or avoid that particular condition.

### Combination treatments and monotherapy

The term "treatment" includes "combination" treatments and therapies, in which two or more treatments or therapies are combined, for example, sequentially or simultaneously. These may be symptomatic or disease modifying treatments.

The particular combination would be at the discretion of the physician.

In combination treatments, the agents (i.e., an MT compound as described herein, plus one or more other agents) may be administered simultaneously or sequentially, and may be administered in individually varying dose schedules and via different routes. For example, when administered sequentially, the agents can be administered at closely spaced intervals (e.g., over a period of 5-10 minutes) or at longer intervals (e.g., 1, 2, 3, 4 or more hours apart, or even longer periods apart where required), the precise dosage regimen being commensurate with the properties of the therapeutic agent(s).

In some embodiments the present invention may be used in combination with oxygen therapy.

In some embodiments the present invention may be used in combination with a further activate agent appropriate to a disease or pathology causing or resulting from hypoxaemia or hypoxia.

In other embodiments the treatment is a "monotherapy", which is to say that the MT-containing compound is not used in combination (within the meaning discussed above) with another active agent.

### Duration of treatment

For treatment of hypoxemia, a treatment regimen based on the MT compounds described herein will preferably extend over a sustained period of time appropriate to the disease and symptoms. The particular duration would be at the discretion of the physician.

For example, the duration of treatment may be:
1 to 14, e.g. 1, 2, 3, 4, 5, 6, 7, 8, 9, 10, 11, 12, 13 or 14 days.
1 to 4, e.g. 1, 2, 3 or 4 weeks.

In all cases the treatment duration will generally be subject to advice and review of the physician.

### Pharmaceutical dosage forms

The MT compound of the invention, or pharmaceutical composition comprising it, may be administered to the stomach of a subject/patient orally (or via a nasogastric tube).

Typically, in the practice of the invention the compound will be administered as a composition comprising the compound, and a pharmaceutically acceptable carrier or diluent.

In some embodiments, the composition is a pharmaceutical composition (e.g., formulation, preparation, medicament) comprising a compound as described herein, and a pharmaceutically acceptable carrier, diluent, or excipient.

The term "pharmaceutically acceptable," as used herein, pertains to compounds, ingredients, materials, compositions, dosage forms, etc., which are, within the scope of sound medical judgment, suitable for use in contact with the tissues of the subject in question (e.g., human) without excessive toxicity, irritation, allergic response, or other problem or complication, commensurate with a reasonable benefit/risk ratio. Each carrier, diluent, excipient, etc. must also be "acceptable" in the sense of being compatible with the other ingredients of the formulation.

In some embodiments, the composition is a pharmaceutical composition comprising at least one compound, as described herein, together with one or more other pharmaceutically acceptable ingredients well known to those skilled in the art, including, but not limited to, pharmaceutically acceptable carriers, diluents, excipients, adjuvants, fillers, buffers, preservatives, anti-oxidants, lubricants, stabilisers, solubilisers, surfactants (e.g., wetting agents), masking agents, colouring agents, flavouring agents, and sweetening agents.

In some embodiments, the composition further comprises other active agents, for example, other therapeutic or prophylactic agents.

Suitable carriers, diluents, excipients, etc. can be found in standard pharmaceutical texts. See, for example, Handbook of Pharmaceutical Additives, 2nd Edition (eds. M. Ash and I. Ash), 2001 (Synapse Information Resources, Inc., Endicott, New York, USA), Remington's Pharmaceutical Sciences, 20th edition, pub. Lippincott, Williams & Wilkins, 2000; and Handbook of Pharmaceutical Excipients, 2nd edition, 1994.

One aspect of the present invention utilises a dosage unit (e.g., a pharmaceutical tablet or capsule) comprising an MT compound as described herein (e.g., obtained by, or obtainable by, a method as described herein; having a purity as described herein; etc.), and a pharmaceutically acceptable carrier, diluent, or excipient.

The "MT compound", although it may be present in relatively low amount, is the active agent of the dosage unit, which is to say is intended to have the therapeutic or prophylactic effect in respect of hypoxemia. Rather, the other ingredients in the dosage unit will be therapeutically inactive e.g. carriers, diluents, or excipients.

Thus, preferably, there will be no other active ingredient in the dosage unit, no other agent intended to have a therapeutic or prophylactic effect in respect of a disorder for which the dosage unit is intended to be used, other than in relation to the combination treatments described herein.

In some embodiments, the dosage unit is a tablet.

In some embodiments, the dosage unit is a capsule.

In some embodiments, said capsules are gelatine capsules.

In some embodiments, said capsules are HPMC (hydroxypropylmethylcellulose) capsules.

The appropriate quantity of MT in the composition will depend on how often it is taken by the subject per day, or how many units are taken at one time. Therefore dosage units may individually contain less than the total daily dose.

An example dosage unit may contain 0.5 to 250 mg of MT.

In some embodiments, the amount is about 1, 2, 3, 4, 5, 6, 7, 8, 9, 10, 11, 12, 13, 14, 15, 16, 17, 18, 19, 20, 30, 40, 50, 60, 70, 80, 90, 100, 110, 120 mg of MT.

Using the weight factors described or explained herein, one skilled in the art can select appropriate amounts of an MT-containing compound to use in oral formulations.

As explained above, the MT weight factor for LMTM is 1.67. Since it is convenient to use unitary or simple fractional amounts of active ingredients, non-limiting example LMTM dosage units may include 17 mg etc.

In one embodiment there is provided a dosage unit pharmaceutical composition which comprises about 17, 27, 34, 51 mg etc. of LMTM.

### Labels, instructions and kits of parts

The unit dosage compositions described herein (LMTX compound plus optionally other ingredients) may be provided in a labelled packet along with instructions for their use.

In one embodiment, the pack is a bottle, such as are well known in the pharmaceutical art. A typical bottle may be made from pharmacopoeial grade HDPE (High-Density Polyethylene) with a childproof, HDPE pushlock closure and contain silica gel desiccant, which is present in sachets or canisters. The bottle itself may comprise a label, and be packaged in a cardboard container with instructions for us and optionally a further copy of the label.

In one embodiment, the pack or packet is a blister pack (preferably one having aluminium cavity and aluminium foil) which is thus substantially moisture-impervious. In this case the pack may be packaged in a cardboard container with instructions for us and label on the container.

Said label or instructions may provide information regarding treatment of hypoxemia.

### Methods of Treatment

Another aspect of the present invention, as explained above, pertains to a method of treatment of hypoxemia comprising administering to a patient in need of treatment a prophylactically or therapeutically effective amount of a compound as described herein, preferably in the form of a pharmaceutical composition.

### Use in Methods of Therapy

Another aspect of the present invention pertains to a compound or composition as described herein, for use in a method of treatment of hypoxemia of the human or animal body by therapy.

### Use in the Manufacture of Medicaments

Another aspect of the present invention pertains to use of an MT compound or composition as described herein, in the manufacture of a medicament for use in treatment of hypoxemia.

In some embodiments, the medicament is a composition e.g. a dose composition as described herein.

### Mixtures of oxidised and reduced MT compounds

The LMT-containing compounds utilised in the present invention may include oxidised (MT⁺) compounds as 'impurities' during synthesis, and may also oxidize (e.g., autoxidize) after synthesis to give the corresponding oxidized forms. Thus, it is likely, if not inevitable, that compositions comprising the compounds of the present invention will contain, as an impurity, at least some of the corresponding oxidized compound. For example an "LMT" salt may include up to 15% e.g. 10 to 15% of MT⁺ salt.

When using mixed MT compounds, the MT dose can be readily calculated using the molecular weight factors of the compounds present.

### Salts and solvates

Although the MT-containing compounds described herein are themselves salts, they may also be provided in the form of a mixed salt (i.e., the compound of the invention in combination with another salt). Such mixed salts are intended to be encompassed by the term "and pharmaceutically acceptable salts thereof". Unless otherwise specified, a reference to a particular compound also includes salts thereof.

The compounds of the invention may also be provided in the form of a solvate or hydrate. The term "solvate" is used herein in the conventional sense to refer to a complex of solute (e.g., compound, salt of compound) and solvent. If the solvent is water, the solvate may be conveniently referred to as a hydrate, for example, a mono-hydrate, a di-hydrate, a tri-hydrate, a penta-hydrate etc. Unless otherwise specified, any reference to a compound also includes solvate and any hydrate forms thereof.

Naturally, solvates or hydrates of salts of the compounds are also encompassed by the present invention.

***

Throughout this specification, including the claims which follow, unless the context requires otherwise, the word "comprise," and variations such as "comprises" and "comprising," will be understood to imply the inclusion of a stated integer or step or group of integers or steps but not the exclusion of any other integer or step or group of integers or steps.

It must be noted that, as used in the specification and the appended claims, the singular forms "a," "an," and "the" include plural referents unless the context clearly dictates otherwise. Thus, for example, reference to "a pharmaceutical carrier" includes mixtures of two or more such carriers, and the like.

Ranges are often expressed herein as from "about" one particular value, and/or to "about" another particular value. When such a range is expressed, another embodiment includes from the one particular value and/or to the other particular value. Similarly, when values are expressed as approximations, by the use of the antecedent "about," it will be understood that the particular value forms another embodiment.

Any sub-titles herein are included for convenience only, and are not to be construed as limiting the disclosure in any way.

The invention will now be further described with reference to the following non-limiting Figures and Examples. Other embodiments of the invention will occur to those skilled in the art in the light of these.

### Figures

Figure 1: oxygen saturation levels in patients receiving LMTX compared pre-dose and after 4 hrs in the clinic following administration of a single doses of LMT at 4 mg and ~100mg (mean of 75mg, 100mg, 125mg). Levels were measured pre-dose and 4 hrs after dosing (post-dose).
Figure 2: the effects of LMTM on SpO2 levels over 4 hours was independent of any corresponding effect on metHb
Figure 3: LMTM at high dosages over a period of time systematically increases metHb levels.
Figure 4: computational chemistry modelling of the high affinity LMT/MT⁺-heme interaction.

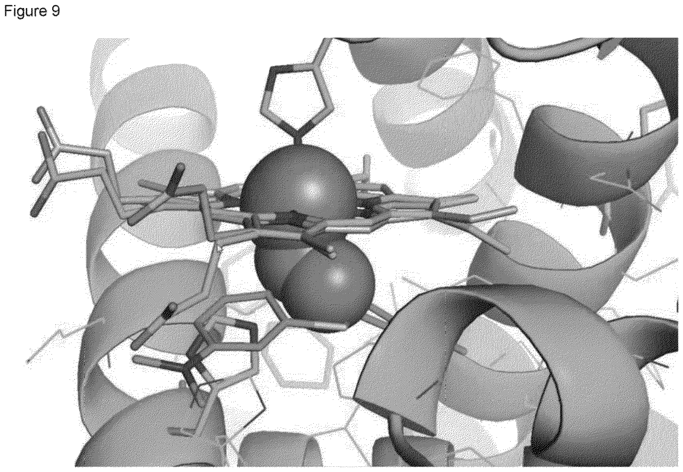

Figures 5-9: illustrations of proposed mechanism of action for LMT in enhancing O₂ binding by haemoglobin, as explained in Example 4 hereinafter.

### Example 1 - Methylthioninium chloride (MTC) and LMTX

MTC (methylthioninium chloride, methylene blue) has been available as a drug since 1876. It is on the world health organisation's list of essential medicines, which is a list of the safest and most effective medicines in a health system.

MTC has been applied previously in many areas of clinical medicine including treatment of methemoglobinemia, malaria, nephrolithiasis, bipolar disorder, ifosfamide encephalopathy and most recently in Alzheimer disease (AD; Wischik et al., 2015; Nedu et al 2020).

The MT moiety can exist in the oxidised MT⁺ form and in the reduced LMT form (Harrington et al., 2015;).

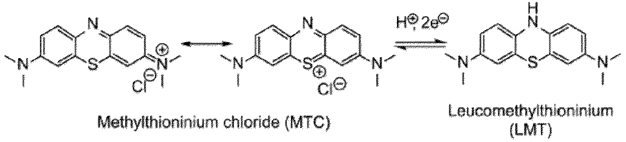

MTC is the chloride salt of the oxidised MT⁺ form. It needs to be converted to the reduced leuco-MT (LMT; international non-proprietary name: hydromethylthionine) form by a thiazine dye reductase activity in the gut to permit absorption and distribution to deep compartments including red cells and brain (Baddeley et al., 2015). Likewise, in isolated red cell preparations, MT⁺ needs to be converted to LMT to permit uptake both into red cells (May et al., 2004) and into pulmonary endothelial cells (Merker et al., 1997).

Because MTC is actually a prodrug for LMT, the predominant form in the body, TauRx developed a stabilised reduced form of MT as LMTM (leuco-methylthioninium bis(hydromethanesulphonate); hydromethylthionine mesylate) in order to permit direct administration of the LMT form.

Synthesis of LMTX and LMTM compounds can be performed according to the methods described in the art (see e.g. WO2007/110627, and WO2012/107706)

### Example 2 - LMTX enhances oxygen saturation in a clinical trial

The present inventors have used data available for patients participating in clinical trials to determine whether LMT enhances oxygen saturation of blood. Data were available for 18 subjects with oxygen saturation < 94% at baseline (lower limit of normal range is 95%).

These patients recorded a variety of respiratory or other conditions of various degrees of severity which it was suspected may have contributed to the detected hypoxemia, including sleep apnoea, insomnia (which may be indicative of Paroxysmal nocturnal dyspnea or paroxysmal nocturnal dyspnoea), asbestosis, oedema, asthma, bronchitis, allergies, angioedema, pneumonia, acute myocardial infarction/ hypertension, Coronary artery disease with angioplasty and stent insertion, transient ischaemic attacks (TIA), hypothyroidism, diabetes, syncope, tachycardia and sepsis.

This is shown in in Table 1:

**Table 1: Presenting clinical history of clinical trial subjects presenting with low oxygen saturation.**

| Subject | Clinical respiratory/ventilation type | Aggravating clinical conditions ¹ |
|---|---|---|
| 1 | Sleep Apnoea (no date given) | hypothyroid / diabetic |
| 2 | Insomnia (no date given) | Often a sign of obstructive sleep apnoea or other mild hypoxia conditions such as paroxysmal nocturnal dyspnea |
| 3 | | Hypertension / LBBB, left bundle branch block; LVH, Left ventricular hypertrophy. |
| 4 | Asbestosis (no date given) | |
| 5 | Oedema (no date given) | Often a sign of right heart failure or congestive cardiac failure / may also be simple sedentary dependent oedema |
| 6 | | Hypertension / LBBB / LVH |
| 7 | | Acute myocardial infarction / Hypertension |
| 8 | | Hypertension |
| 9 | | Hypertension / Coronary artery disease with angioplasty and stent insertion |
| 10² | | |
| 11² | | |
| 12 | Asthma - childhood | |
| | Sleep apnoea with uvulectomy (2006) | |
| 13 | Bronchitis (2007) | |
| | Seasonal allergies (2009) | |
| | Anaemia (2012) | |
| 14 | | Hypertension / Carotid artery disease with stent insertion (1996) |
| 15 | | Acute myocardial infarction with stent insertion (2011) |
| 16² | | |
| 17 | | Transient ischaemic attack (2004) / Hypertension (2012) |
| 18 | Asthma - childhood | Hypertension / hypothyroid / syncope / tachycardia / sepsis |
| | Intermittent angioedema (2013) | |
| | Occassional insomnia (no date) | |
| | Pneumonia (no date) | |

| | | |
|---|---|---|
| ¹ Aggravating clinical conditions that may also cause hypoxia if sufficiently severe or chronic. ² Three patients had no predisposing clinical history factors listed. | | |

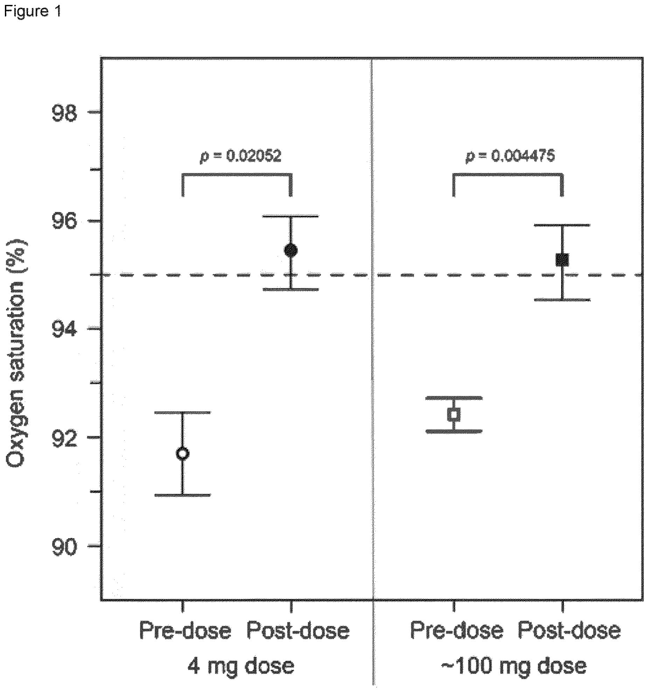

Oxygen saturation levels were compared pre-dose and after 4 hrs in the clinic following administration of a single doses of LMT at 4 mg and ~100mg (mean of 75, 100, 125 mg; Fig 1).

LMTM at both dosing ranges significantly increased oxygen saturation at 4 hours, again supporting multiple beneficial modes of action for LTMX for treatment of COVID-19 patients.

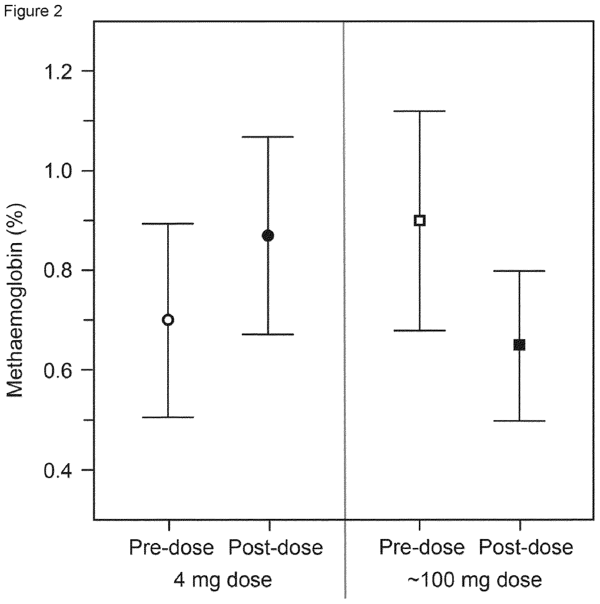

In order to understand this effect further the inventors investigated whether the low oxygen saturation in these patients is due to elevation in metHb levels. There was no difference in metHb levels at baseline between subjects with low SpO₂ and those with SpO₂ levels in the normal range. Furthermore, the effects on LMTM on SpO₂ levels over 4 hours was independent of any corresponding effect on metHb (Fig 2).

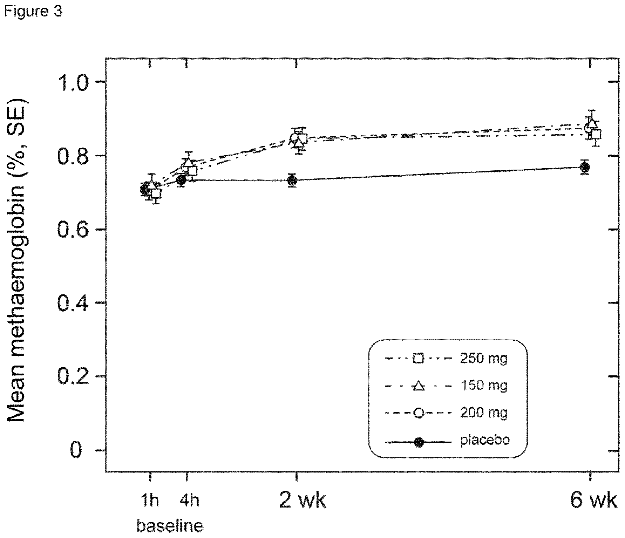

Therefore, LMTM is able to act on haemoglobin over a range of doses in such a way as to enhance oxygen saturation in the blood by a novel mechanism unrelated to its known effects on metHb. Indeed LMTM at higher doses systematically increases metHb levels (Fig 3).

### Example 3 - MT and methemoglobinemia

Methemoglobinemia is the result of oxidation of the iron contained in haemoglobin from the ferrous (Fe²⁺) to the ferric (Fe³⁺) form. The oxidation is associated with a decrement in the capacity of haemoglobin to carry oxygen efficiently (Curry et al., 1982). This is because the binding of oxygen to metHb is irreversible in a given heamoglobin subunit. However binding in one of the four units of the haemoglobin tetramer enhances oxygen binding affinity in other members via structural changes in globin (the mechanism of co-operativity). This results in an overall increase in oxygen binding affinity and in increase in oxygen saturation, or a left shift in the oxygen-haemoglobin saturation curve. Because binding of di-oxygen to heme iron is irreversible, there is a reduced capacity for haemoglobin to release oxygen to hypoxic tissues. This results in net tissue hypoxia without a reduction in SpO₂.

MTC is the primary treatment for methemoglobinemia, and indeed represents the only approved indication for its clinical use. The oxidised MT⁺ form of methylthionine given as MTC is first reduced to LMT at the cell surface as a prerequisite for red cell entry (May et al., 2004). It is then LMT which is the active species at the heme site, forming a coordinate with heme iron and permitting the transfer of an electron which converts Fe³⁺ to Fe²⁺. This restores normal oxygen-carrying capacity (Yubisui et al., 1980; Blank et al., 2012). This is therefore a redox reaction which results in oxidation of LMT to MT⁺. LMT is regenerated from MT⁺ via a redox reaction with NADPH which is itself regenerated from NADP by way of ongoing glycolysis in the red cell. In conditions which exceed the red cell's reducing capacity (e.g. high doses of LMTX or a G6PD deficiency which impairs the efficiency of glycolysis), LMTX can induce methaemoglobinaemia. In both cases, the LMT moiety is acting as an electron shuttle within the red cell, as it does also in other systems (e.g. in the electron transport chain in mitochondria).

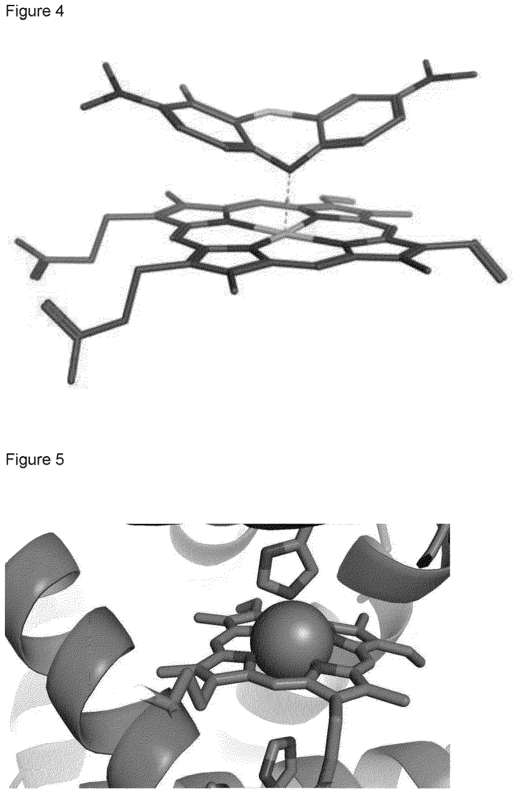

Computational chemistry modelling shown in Fig 4 provides a structural basis explaining the dynamics of the high affinity LMT/MT⁺-heme interaction. The LMT nitrogen orientates itself towards the Fe³⁺ of the heme porphyrin within 2.1Å (dotted line in Figure 1). This close interaction then facilitates the transfer of an electron from LMT to the Fe³⁺, thereby reducing it to Fe²⁺ and the resulting formation of MT⁺. Conversely, in conditions of impaired glycolysis or high levels of LMT/MT, the same binding interaction with heme permit the transfer of an electron from Fe²⁺ to MT⁺ producing Fe³⁺ and LMT respectively.

Given that the LMT is the active form, the clinical evidence above indicates that this LMT-heme interaction facilitates oxygen uptake by haemoglobin. Conversely, the available clinical evidence also shows that LMT at high concentrations (associated with oral doses in the range 150 - 250 mg/day) can produce a measurable increase in metHb levels, yet at the same time also increase SpO₂ levels. It therefore follows that the effects on LMT on SpO₂ cannot be mediated via effects on metHb levels.

### Example 4 - proposed mechanism for SpO₂ effect of LMT

Without wishing to be bound by theory, the inventors propose the possible mechanism for the observed clinical evidence described herein.

When Hb is in the deoxygenated state, the heme is in the domed T state with Fe not fully accommodated in the tetrapyrrole ring, and is held by two histidines (His 87 in alpha subunit / His 92 in beta subunit and His 58 in alpha subunit / His 63 in beta submit). In this state, the ionic radius of the iron, which is in a high-spin Fe(ll) state, is too large (radius 2.06Å) to fit in the ring of nitrogens with which it coordinates; it is 0.6Å out of the plane of the ring (Fig 5).

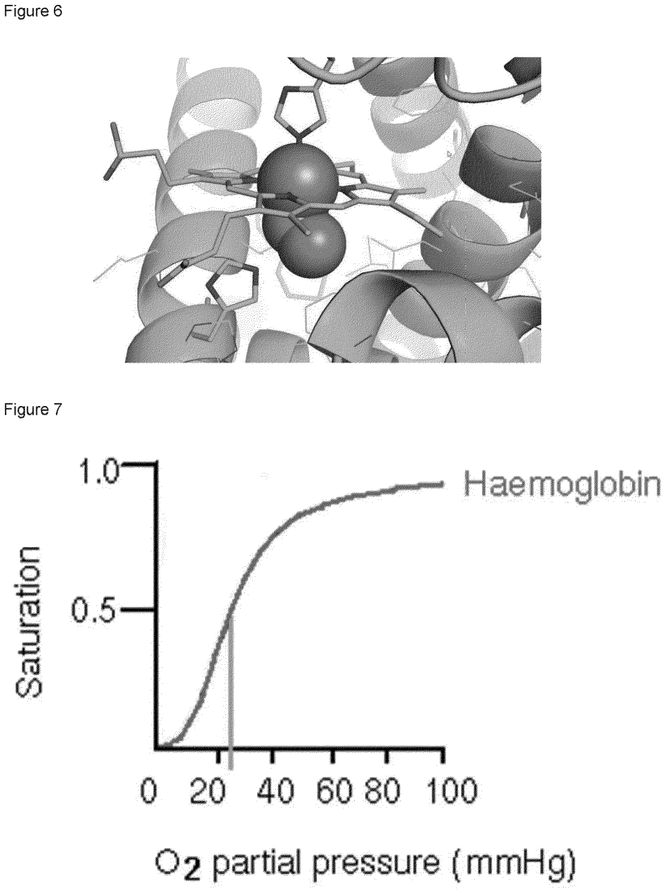

When O₂ binds to the heme group it assumes the R state, becomes planar and the iron ion lies in the plane of the ring, as it is in a low-spin Fe(II) state with a smaller radius (1.98Å). All six coordination positions of the ion are occupied: the bound oxygen molecule accounts for the sixth. When O₂ binds to Fe²⁺, it displaces the distal histidine and stabilises the heme moiety in the flat R-state (Fig 6).

The binding of oxygen by haemoglobin is cooperative. As haemoglobin binds successive oxygens, the oxygen affinity of the subunits increases. The affinity for the fourth oxygen to bind is approximately 300 times that for the first. The result is the sigmoid oxygen saturation curve (Fig 7).

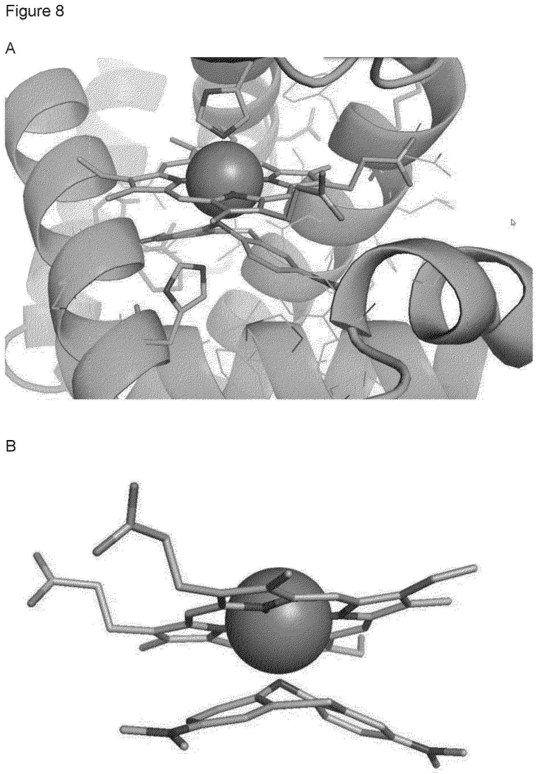

MT is estimated to bind to the Fe of heme with an estimated field factor of 1.2 - 1.5. LMT binds with high affinity. The field factor of LMT is sufficient to bind to Fe²⁺ (potentially f-factor of 1.2-1.5; CK Jorgensen, Oxidation numbers and oxidation states, Springer 1969 pp84-85). MT is therefore a strong field ligand and is able to bind to heme sufficiently to induce an R-state configuration within the protein (Fig 8A & 8B). The MT moiety is able to form a complex with Fe²⁺ by donation of lone pair electrons from the N atom to the d-orbitals of ferrous iron (Molecules 2013, 18(3), 3168-3182; https://doi.org/10.3390/molecules18033168)

Therefore, binding of LMT overcomes the initial energy barrier for oxygen binding, which is thereafter able to bind and oxygenate all four heme groups of haemoglobin.

The Fe²⁺ ion coordination complex is filled by binding to four nitrogen atoms in the pyrrole rings, and a fifth ligand is a supplied by the proximal Histidine of haemoglobin. In the absence of O₂, the sixth coordination ligand is vacant, and the geometry of the complex is square pyramidal with the Fe²⁺ above the plane of the heme ring resulting in the characteristic domed geometry of the deoxy T-state. Upon O₂ binding into the sixth coordination site, this results in the Fe²⁺ into the plane of the ring, leading to octahedral geometry. LMT is likely to induce a transition to the flat R-state and hence facilitates oxygen binding by way of the co-operativity mechanism. However, the LMT binding is non-optimal and produces a subtle conformational change in haemoglobin which potentially perturbs the orientation of the octahedral coordination complex from the optimal geometry. The non-optimal geometry of the LMT coordination compared to oxygen results in oxygen binding heme with higher affinity than LMT. Whereas the binding distance between the LMT nitrogen and heme iron is 2.10Å, the corresponding binding distance for oxygen is it is 1.98Å. Therefore, oxygen is able to displace LMT when it is available at high pH / low pCO₂. This permits normal oxygen dissociation to occur with release of bound oxygen to peripheral tissues at low pH / high pCO₂ (Fig (9))..

### References

Atamna, H., & Kumar, R. (2010). Protective role of methylene blue in Alzheimer's disease via mitochondria and cytochrome c oxidase. In Journal of Alzheimer's Disease (Vol. 20, Issue SUPPL.2). https://doi.org/10.3233/JAD-2010-100414
Baddeley, T. C., McCaffrey, J., M. D. Storey, J., Cheung, J. K. S., Melis, V., Horsley, D., Harrington, C. R., & Wischik, C. M. (2015). Complex Disposition of Methylthioninium Redox Forms Determines Efficacy in Tau Aggregation Inhibitor Therapy for Alzheimer's Disease. Journal of Pharmacology and Experimental Therapeutics, 352(1), 110-118. https://doi.org/10.1124/jpet.114.219352
Baig, A. M., Khaleeq, A., Ali, U., & Syeda, H. (2020). Evidence of the COVID-19 Virus Targeting the CNS: Tissue Distribution, Host-Virus Interaction, and Proposed Neurotropic Mechanisms. In ACS Chemical Neuroscience (Vol. 11, Issue 7, pp. 995-998). https://doi.org/10.1021/acschemneuro.0c00122
Blank, O., Davioud-Charvet, E., & Elhabiri, M. (2012). Interactions of the Antimalarial Drug Methylene Blue with Methemoglobin and Heme Targets in Plasmodium falciparum : A Physico-Biochemical Study. Antioxidants & Redox Signaling, 17(4), 544-554. https://doi.org/10.1089/ars.2011.4239
Bojadzic, D., Alcazar, O., & Buchwald, P. (2020). Methylene Blue Inhibits In Vitro the SARS-CoV-2 Spike-ACE2 Protein-Protein Interaction-A Mechanism That Can Contribute to Its Antiviral Activity Against COVID-19. BioRxiv, 2020.08.29.273441. https://doi.org/10.1101/2020.08.29.273441
Cagno, V., Medaglia, C., Cerny, A., Cerny, T., & Cerny, E. (2020). Methylene Blue has a potent antiviral activity against SARS-CoV-2 in the absence of UV-activation in vitro. BioRxiv, 2020.08.14.251090. https://doi.org/10.1101/2020.08.14.251090
Curry S. Methemoglobinemia. Ann Emerg Med. 1982. 2:214-21
De Felice, F. G., Tovar-Moll, F., Moll, J., Munoz, D. P., & Ferreira, S. T. (2020). Severe Acute Respiratory Syndrome Coronavirus 2 (SARS-CoV-2) and the Central Nervous System. In Trends in Neurosciences (Vol. 43, Issue 6, pp. 355-357). https://doi.org/10.1016/j.tins.2020.04.004
de la Vega, M. R., Dodson, M., Gross, C., Mansour, H. M., Lantz, R. C., Chapman, E., Wang, T., Black, S. M., Garcia, J. G. N., & Zhang, D. D. (2016). Role of Nrf2 and Autophagy in Acute Lung Injury. In Current Pharmacology Reports (Vol. 2, Issue 2, pp. 91-101). https://doi.org/10.1007/s40495-016-0053-2
Gureev, A. P., Syromyatnikov, M. Y., Gorbacheva, T. M., Starkov, A. A., & Popov, V. N. (2016). Methylene blue improves sensorimotor phenotype and decreases anxiety in parallel with activating brain mitochondria biogenesis in mid-age mice. Neuroscience Research, 113, 19-27. https://doi.org/10.1016/j.neures.2016.07.006
Guzzi, P. H., Mercatelli, D., Ceraolo, C., & Giorgi, F. M. (2020). Master Regulator Analysis of the SARS-CoV-2/Human Interactome. Journal of Clinical Medicine, 9(4), 982. https://doi.org/10.3390/jcm9040982
Harrington, C. R., Storey, J. M. D., Clunas, S., Harrington, K. A., Horsley, D., Ishaq, A., Kemp, S. J., Larch, C. P., Marshall, C., Nicoll, S. L., Rickard, J. E., Simpson, M., Sinclair, J. P., Storey, L. J., & Wischik, C. M. (2015). Cellular Models of Aggregation-dependent Template-directed Proteolysis to Characterize Tau Aggregation Inhibitors for Treatment of Alzheimer Disease. Journal of Biological Chemistry, 290(17), 10862-10875. https://doi.org/10.1074/jbc.M114.616029
May, J. M., Qu, Z. C., & Cobb, C. E. (2004). Reduction and uptake of methylene blue by human erythrocytes. American Journal of Physiology - Cell Physiology, 286(6 55-6). https://doi.org/10.1152/ajpcell.00512.2003
Mehta G, Mawdsley A et al., the effect of oral methylene blue on viral load in chronic hepatitis C infection. Poster presented at British association for the study of the liver (BASL) meeting. 2006 Sept. Dublin, Ireland.
Melchinger, H., Jain, K., Tyagi, T., & Hwa, J. (2019). Role of Platelet Mitochondria: Life in a Nucleus-Free Zone. Frontiers in Cardiovascular Medicine, 6. https://doi.org/10.3389/fcvm.2019.00153
Merker, M. P., Bongard, R. D., Linehan, J. H., Okamoto, Y., Vyprachticky, D., Brantmeier, B. M., Roerig, D. L., & Dawson, C. A. (1997). Pulmonary endothelial thiazine uptake: Separation of cell surface reduction from intracellular reoxidation. American Journal of Physiology - Lung Cellular and Molecular Physiology, 272(4 16-4). https://doi.org/10.1152/ajplung.1997.272.4.I673
Mohr, H., Bachmann, B., Klein-Struckmeier, A., & Lambrecht, B. (1997). Virus inactivation of blood products by phenothiazine dyes and light. Photochemistry and Photobiology, 65(3), 441-445. https://doi.org/10.1111/j.1751-1097.1997.tb08586.x
Müller-Breitkreutz, K., & Mohr, H. (1998). Hepatitis C and human immunodeficiency virus RNA degradation by methylene blue/light treatment of human plasma. Journal of Medical Virology, 56(3), 239-245. https://doi.org/10.1002/(SICI)1096-9071(199811)56:3<239::AID-JMV11>3.0.CO;2-9
Naymagon, L., Berwick, S., Kessler, A., Lancman, G., Gidwani, U., & Troy, K. (2020). The emergence of methemoglobinemia amidst the COVID-19 pandemic. Am J Hematol, 95, E196-E19. https://doi.org/10.1002/ajh.25868
Nedu, M. E., Tertis, M., Cristea, C., & Georgescu, A. V. (2020). Comparative study regarding the properties of methylene blue and proflavine and their optimal concentrations for in vitro and in vivo applications. In Diagnostics (Vol. 10, Issue 4). https://doi.org/10.3390/diagnostics10040223
Ramani, A., Müller, L., Ostermann, P. N., Gabriel, E., Abida-Islam, P., Müller-Schiffmann, A., Mariappan, A., Goureau, O., Gruell, H., Walker, A., Andrée, M., Hauka, S., Houwaart, T., Dilthey, A., Wohlgemuth, K., Omran, H., Klein, F., Wieczorek, D., Adams, O., ... Gopalakrishnan, J. (2020). SARS-CoV-2 targets cortical neurons of 3D human brain organoids and shows neurodegeneration-like effects. BioRxiv (Preprint), 2020.05.20.106575. https://doi.org/10.1101/2020.05.20.106575
Riedel, G., Klein, J., Niewiadomska, G., Kondak, C., Schwab, K., Lauer, D., Magbagbeolu, M., Steczkowska, M., Zadrozny, M., Wydrych, M., Cranston, A., Melis, V., Santos, R. X., Theuring, F., Harrington, C. R., & Wischik, C. M. (2020). Mechanisms of Anticholinesterase Interference with Tau Aggregation Inhibitor Activity in a Tau-Transgenic Mouse Model. Current Alzheimer Research, 17(3), 285-296. https://doi.org/10.2174/1567205017666200224120926
Rodriguez, P., Jiang, Z., Huang, S., Shen, Q., & Duong, T. Q. (2014). Methylene blue treatment delays progression of perfusion-diffusion mismatch to infarct in permanent ischemic stroke. Brain Research, 1588, 144-149. https://doi.org/10.1016/j.brainres.2014.09.007
Saleh, J., Peyssonnaux, C., Singh, K.C., & Edeas, M. (2020). Mitochondria and microbiota dysfunction in COVID-19 pathogenesis. Mitochondrion, 54, 1-7. https://doi.org/10.1016/j.mito.2020.06.008
Schelter, B. O., Shiells, H., Baddeley, T. C., Rubino, C. M., Ganesan, H., Hammel, J., Vuksanovic, V., Staff, R. T., Murray, A. D., Bracoud, L., Riedel, G., Gauthier, S., Jia, J., Bentham, P., Kook, K., Storey, J. M. D., Harrington, C. R., & Wischik, C. M. (2019). Concentration-Dependent Activity of Hydromethylthionine on Cognitive Decline and Brain Atrophy in Mild to Moderate Alzheimer's Disease. Journal of Alzheimer's Disease, 72(3), 931-946. https://doi.org/10.3233/JAD-190772
Singh, K. K., Chaubey, G., Chen, J. Y., & Suravajhala, P. (2020). Decoding sars-cov-2 hijacking of host mitochondria in covid-19 pathogenesis. In American Journal of Physiology - Cell Physiology (Vol. 319, Issue 2, pp. C258-C267). https://doi.org/10.1152/ajpcell.00224.2020
Stack, C., Jainuddin, S., Elipenahli, C., Gerges, M., Starkova, N., Starkov, A. A., Jové, M., Portero-Otin, M., Launay, N., Pujol, A., Kaidery, N. A., Thomas, B., Tampellini, D., Flint Beal, M., & Dumont, M. (2014). Methylene blue upregulates Nrf2/ARE genes and prevents tau-related neurotoxicity. Human Molecular Genetics, 23(14), 3716-3732. https://doi.org/10.1093/hmg/ddu080
Wang, M., Cao, R., Zhang, L., Yang, X., Liu, J., Xu, M., Shi, Z., Hu, Z., Zhong, W., & Xiao, G. (2020). Remdesivir and chloroquine effectively inhibit the recently emerged novel coronavirus (2019-nCoV) in vitro. Cell Res, 30(3), 269-271. https://doi.org/10.1038/s41422-020-0282-0
Wischik, C. M., Edwards, P. C., Lai, R. Y. K., Roth, M., & Harrington, C. R. (1996). Selective inhibition of Alzheimer disease-like tau aggregation by phenothiazines. Proceedings of the National Academy of Sciences, 93(20), 11213-11218. https://doi.org/10.1073/pnas.93.20.11213
Wischik, C. M., Staff, R. T., Wischik, D. J., Bentham, P., Murray, A. D., Storey, J. M. D., Kook, K. A., & Harrington, C. R. (2015). Tau Aggregation Inhibitor Therapy: An Exploratory Phase 2 Study in Mild or Moderate Alzheimer's Disease. Journal of Alzheimer's Disease, 44(2), 705-720. https://doi.org/10.3233/JAD-142874
Wood C, Nagy H. Methylene blue therapy of viral disease. US20060264423 A1, United States Patent and Trademark Office, 19 May 2006.
Yao, X., Ye, F., Zhang, M., Cui, C., Huang, B., Niu, P., Liu, X., Zhao, L., Dong, E., Song, C., Zhan, S., Lu, R., Li, H., Tan, W & Liu, D. (2020). In Vitro Antiviral Activity and Projection of Optimized Dosing Design of Hydroxychloroquine for the Treatment of Severe Acute Respiratory Syndrome Coronavirus 2 (SARS-CoV-2). Clin Infect Dis, 71(15), 732-739. https://doi.ora/10.1093/cid/ciaa237
Yubisui T, Takeshita M, Yoneyama Y. Reduction of methemoglobin through flavin at the physiological concentration by NADPH-flavin reductase of human erythrocytes. J Biochem. 1980. 87(6): 1715-20.
Zhou, H., Lu, S., Chen, J., Wei, N., Wang, D., Lyu,H., Shi, C., & Hua, S. (2020). The landscape of cognitive function in recovered COVID-19 patients. J Psychiatr Res. 129, 98-102. https://doi.org/10.1016/j.jpsychires.2020.06.022

## Claims

1. An MT-containing compound for use in a method of alleviating hypoxemia in a subject,
wherein the subject has a blood oxygen saturation level (SpO2) of less than 95% on room air, optionally less than or equal to 94%, 93%, 92%, 91% or 90%,
which method comprises
(i) selecting the subject according to their SpO2 value;
(ii) orally administering to said subject a methylthioninium (MT)-containing compound,
wherein said administration provides a total daily oral dose of 0.5 mg to 250 mg of MT to the subject per day, optionally split into 2 or more doses,
wherein the MT-containing compound has the following formula: 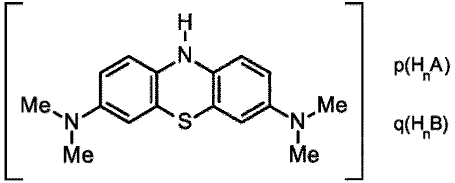
wherein each of HₙA and HₙB (where present) are protic acids which may be the same or different,
and wherein p = 1 or 2; q = 0 or 1; n = 1 or 2; (p + q) × n = 2,
or a hydrate or solvate thereof.

2. The compound for use as claimed in claim 1 wherein the total daily dose is:
(i) greater than 35, 40, 50, or 60 mg and less than or equal to 250 mg of MT to the subject per day; and/or
(ii) greater than or equal to about 30.5, 30.6, 31, 35, 37.5, 40, 45, 50, 55, 60, 65, 70, 75, 80, 85, 90, 95, 100, 105, 110, 115, 120, 125, 130, 140, 150, 160, 170, 180, 200, 210, 220, 230, 240, or 250 mg MT.

3. The compound for use as claimed in claim 1 or claim 2 wherein the total daily dose is about 60, 75, or 120 mg MT.

4. The compound for use as claimed in claim 1 wherein the total daily dose is from about any of 0.5, 1, 1.5, 2, 2.5, 3, 3.5, 4 mg to about any of 5, 6, 7, 8, 9, 10 mg.

5. The compound for use as claimed in any one of claims 1 to 4 wherein the total daily dose of the compound is administered as a split dose twice a day.

6. The compound for use as claimed in any one of claims 1 to 5 wherein the subject is additionally selected from: a hypotensive subject whose systolic BP is less than 80mmHg; a subject in respiratory or cardiac arrest; a neonatal patient in distress; a subject with suspected sickle cell crisis; a subject with carbon monoxide poisoning.

7. The compound for use as claimed in any one of claims 1 to 6 wherein the subject is diagnosed with a disease or pathology resulting in, or arising from, hypoxemia, wherein the disease or pathology is selected from: anaemia; ARDS (Acute respiratory distress syndrome); asbestosis; asthma; bronchitis; carbon monoxide poisoning; cerebral hypoxia; cerebral hypoxia induced by excessive G forces (G-LOC); congenital heart defects in children; congenital heart disease in adults; congestive cardiac failure; COPD (chronic obstructive pulmonary disease); COVID-19; cyanide poisoning; cystic fibrosis; emphysema; histotoxic hypoxia; hypoventilation training; insomnia; intermittent angioedema; interstitial lung disease; intrauterine hypoxia; ischaemic hypoxia; lung injury, caused by trauma or infection, which is optionally bacterial, viral or fungal; adverse response to medication that depresses breathing; pneumonia; pneumothorax; pulmonary oedema; pulmonary embolism; pulmonary fibrosis; pulmonary hypertension; respiratory alkalosis; sleep apnoea; transient ischaemic attack; tuberculosis; tumour hypoxia.

8. The compound for use as claimed in any one of claims 1 to 7 wherein the MT-containing compound is used in combination with supplementary oxygen therapy.

9. The compound for use as claimed in any one of claims 1 to 8 wherein the MT-containing compound has the following formula, where HA and HB are different mono-protic acids:

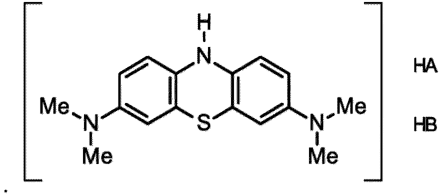

10. The compound for use as claimed in any one of claims 1 to 8 wherein the MT-containing compound has the following formula: 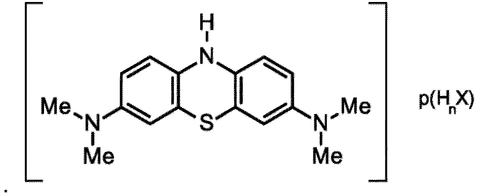 wherein each of HₙX is a protic acid, wherein optionally the MT-containing compound has the following formula and is a bis-monoprotic acid:

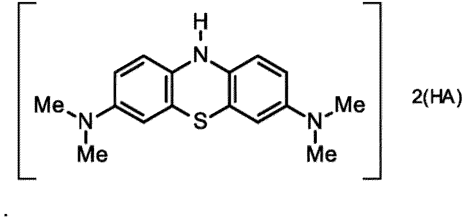

11. The compound for use as claimed in any one of claims 1 to 8 wherein the MT-containing compound has the following formula and H₂A is a di-protic acid:

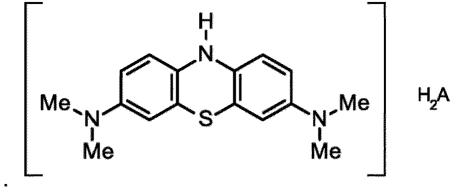

12. The compound for use as claimed in any one of claims 1 to 11 wherein the or each protic acid is an inorganic acid, which is optionally a hydrohalide acid or HNO₃ or H₂SO₄, wherein the hydrohalide acid is optionally selected from HCl; HBr.

13. The compound for use as claimed in any one of claims 1 to 11 wherein the or each protic acid is an organic acid, which is optionally selected from H₂CO₃; CH₃COOH; methanesulfonic acid, 1,2-ethanedisulfonic acid, ethanesulfonic acid, naphthalenedisulfonic acid, p-toluenesulfonic acid.

14. The compound for use as claimed in any one of claims 1 to 11, or claim 13 wherein the MT-containing compound is LMTM:

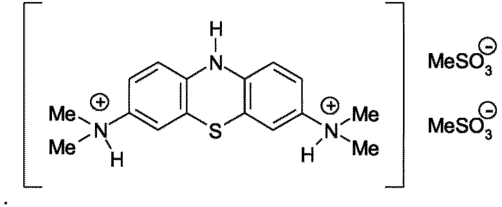

15. The compound for use as claimed in any one of claims 1 to 11 wherein the MT-containing compound is selected from the list consisting of:

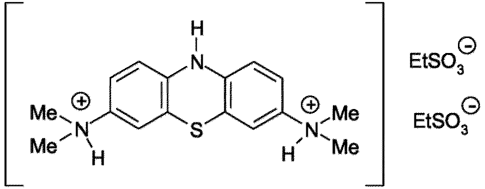

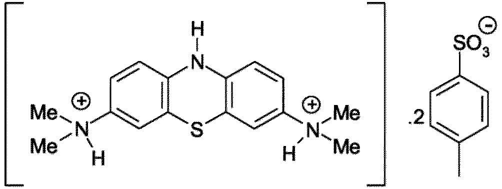

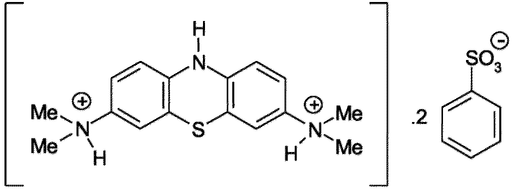

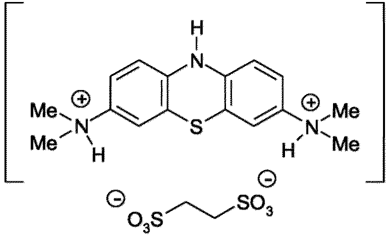

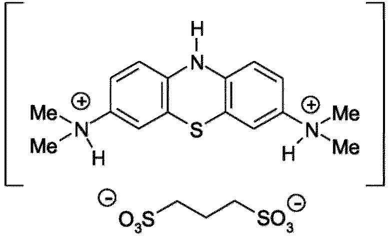

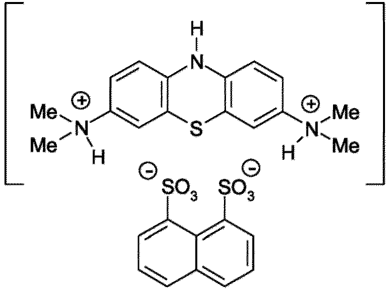

## Patentansprüche

1. MT-hältige Verbindung zur Verwendung in einem Verfahren zum Lindern von Hypoxämie in einem Individuum,
wobei das Individuum einen Blutsauerstoffsättigungsspiegel (SpO2) von weniger als 95 % bei Raumluft, gegebenenfalls weniger oder gleich 94 %, 93 %, 92 %, 91 % oder 90 %, aufweist,
wobei das Verfahren Folgendes umfasst:
(i) Auswählen des Individuums gemäß dem SpO2-Wert;
(ii) orales Verabreichen einer Methylthioninium- (MT-) hältigen Verbindung,
wobei die Verabreichung eine orale tägliche Gesamtdosis von 0,5 mg bis 250 mg MT pro Tag an das Individuum liefert, gegebenenfalls aufgeteilt in 2 oder mehr Dosen,
wobei die MT-hältige Verbindung folgende Formel aufweist: 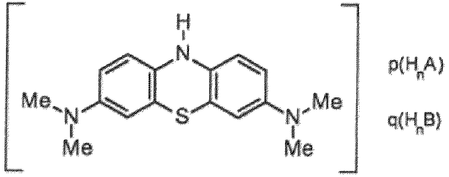
worin jedes aus HₙA und HₙB (falls vorhanden) protische Säuren sind, die gleich oder verschieden sein können,
und wobei p = 1 oder 2 ist; q = 0 oder 1 ist; n = 1 oder 2 ist; (p + q) × n = 2 ist,
oder ein Hydrat oder Solvat davon.

2. Verbindung zur Verwendung nach Anspruch 1, wobei die tägliche Gesamtdosis Folgendes ist:
(i) größer als 35, 40, 50 oder 60 mg und kleiner oder gleich 250 mg MT an das Individuum pro Tag; und/oder
(ii) größer als oder gleich etwa 30,5, 30,6, 31, 35, 37,5, 40, 45, 50, 55, 60, 65, 70, 75, 80, 85, 90, 95, 100, 105, 110, 115, 120, 125, 130, 140, 150, 160, 170, 180, 200, 210, 220, 230, 240 oder 250 mg MT.

3. Verbindung zur Verwendung nach Anspruch 1 oder Anspruch 2, wobei die tägliche Gesamtdosis etwa 60, 75 oder 120 mg MT beträgt.

4. Verbindung zur Verwendung nach Anspruch 1, wobei die tägliche Gesamtdosis von einem beliebigen von 0,5, 1, 1,5, 2, 2,5, 3, 3,5, 4 mg bis etwa einem beliebigen von 5, 6, 7, 8, 9, 10 mg beträgt.

5. Verbindung zur Verwendung nach einem der Ansprüche 1 bis 4, wobei die tägliche Gesamtdosis der Verbindung zweimal täglich als geteilte Dosis verabreicht wird.

6. Verbindung zur Verwendung nach einem der Ansprüche 1 bis 5, wobei das Individuum zusätzlich dazu ausgewählt ist aus: einem hypotensiven Individuum, dessen systolischer BP unter 80 mmHg liegt; einem Individuum mit Atem- oder Herzstillstand; einem neonatalen Notfallpatienten; einem Individuum mit vermuteter Sichelzellkrise; einem Individuum mit Kohlenmonoxidvergiftung.

7. Verbindung zur Verwendung nach einem der Ansprüche 1 bis 6, wobei bei dem Individuum eine Erkrankung oder Pathologie diagnostiziert wurde, die zu Hypoxämie führt oder daraus entsteht, wobei die Erkrankung oder Pathologie aus Folgendem ausgewählt ist: Anämie; ARDS (akutem Atemnotsyndrom); Asbestose; Asthma; Bronchitis; Kohlenmonoxidvergiftung; zerebraler Hypoxie; durch übermäßige G-Kräfte induzierter zerebraler Hypoxie (G-LOC); angeborenem Herzfehler bei Kindern; angeborenen Herzerkrankungen bei Erwachsenen; kongestiver Herzinsuffizienz; COPD (chronisch-obstruktiver Lungenerkrankung); COVID-19; Cyanidvergiftung; zystischer Fibrose; Emphysem; histotoxischer Hypoxie; Hypoventilationstraining; Schlaflosigkeit; intermittierendem Angioödem; interstitieller Lungenerkrankung; intrauteriner Hypoxie; ischämischer Hypoxie; Lungenverletzung durch Trauma oder Infektion, wahlweise bakteriell, viral oder pilzbedingt; unerwünschter Reaktion auf Medikamente, die die Atmung beeinträchtigen; Lungenentzündung; Pneumothorax; Lungenödem; Lungenembolie; Lungenfibrose; pulmonaler Hypertonie; respiratorischer Alkalose; Schlafapnoe; transitorischer ischämischer Attacke; Tuberkulose; Tumorhypoxie.

8. Verbindung zur Verwendung nach einem der Ansprüche 1 bis 7, wobei die MT-hältige Verbindung in Kombination mit ergänzender Sauerstofftherapie verwendet wird.

9. Verbindung zur Verwendung nach einem der Ansprüche 1 bis 8, wobei die MT-hältige Verbindung folgende Formel aufweist, worin HA und HB verschiedene monoprotische Säuren sind:

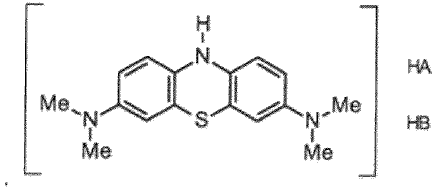

10. Verbindung zur Verwendung nach einem der Ansprüche 1 bis 8, wobei die MT-hältige Verbindung folgende Formel aufweist: 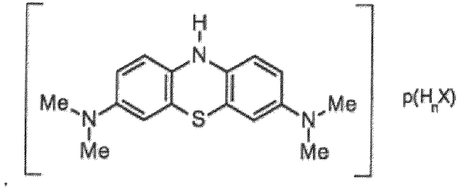 worin jedes der HₙX eine protische Säure ist, wobei die MT-hältige Verbindung gegebenenfalls die folgende Formel aufweist und eine Bis-monoprotische Säure ist:

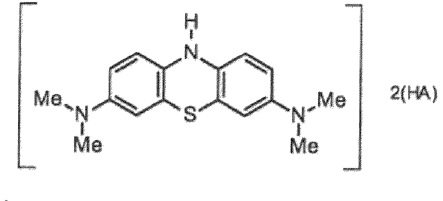

11. Verbindung zur Verwendung nach einem der Ansprüche 1 bis 8, wobei die MT-hältige Verbindung die folgende Formel aufweist und H₂A eine diprotische Säure ist:

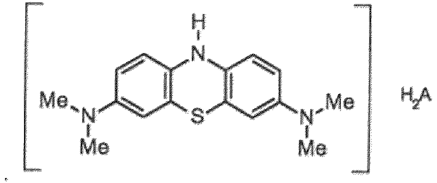

12. Verbindung zur Verwendung nach einem der Ansprüche 1 bis 11, wobei die oder jede protische Säure eine anorganische Säure ist, die gegebenenfalls eine Halogenwasserstoffsäure oder HNO₃ oder H₂SO₄ ist, wobei die Halogenwasserstoffsäure gegebenenfalls aus HCl; HBr ausgewählt ist.

13. Verbindung zur Verwendung nach einem der Ansprüche 1 bis 11, wobei die oder jede protische Säure eine organische Säure ist, die gegebenenfalls aus H₂CO₃; CH₃COOH; Methansulfonsäure, 1,2-Ethandisulfonsäure, Ethansulfonsäure, Naphthalindisulfonsäure, p-Toluolsulfonsäure ausgewählt ist.

14. Verbindung zur Verwendung nach einem der Ansprüche 1 bis 11 oder Anspruch 13, wobei die MT-hältige Verbindung LMTM ist:

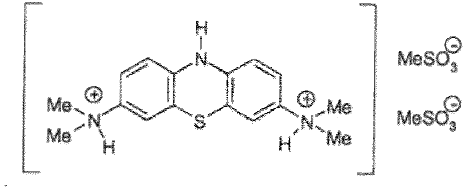

15. Verbindung zur Verwendung nach einem der Ansprüche 1 bis 11, wobei die MT-hältige Verbindung aus der aus Folgenden bestehenden Liste ausgewählt ist:

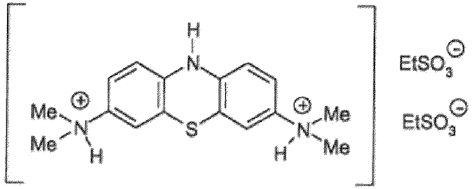

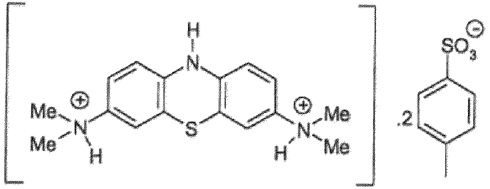

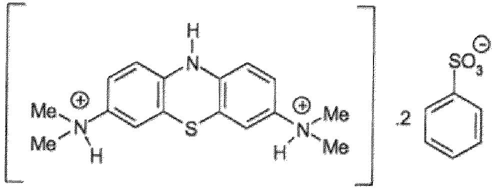

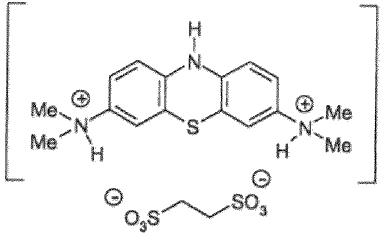

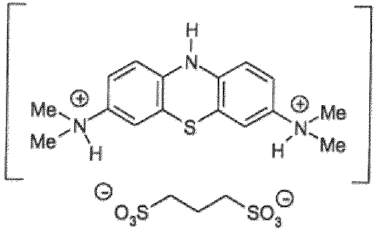

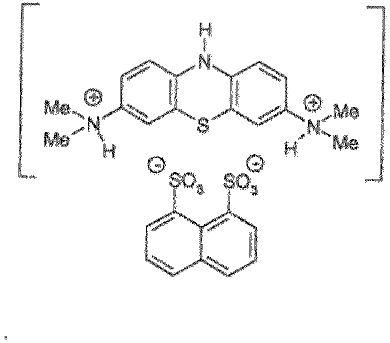

## Revendications

1. Composé contenant du MT à utiliser dans un procédé de soulagement de l'hypoxémie chez un sujet,
dans lequel le sujet présente un taux de saturation en oxygène du sang (SpO2) inférieur à 95 % dans l'air ambiant, facultativement inférieur ou égal à 94 %, 93 %, 92 %, 91 % ou 90 %,
lequel procédé comprenant les étapes consistant à :
(i) sélectionner le sujet en fonction de sa valeur de SpO2 ;
(ii) administrer par voie orale audit sujet un composé contenant du méthylthioninium (MT),
dans lequel ladite administration fournit une dose orale quotidienne totale allant de 0,5 mg à 250 mg de MT par jour au sujet, facultativement divisée en 2 doses ou plus,
dans lequel le composé contenant du MT présente la formule suivante : 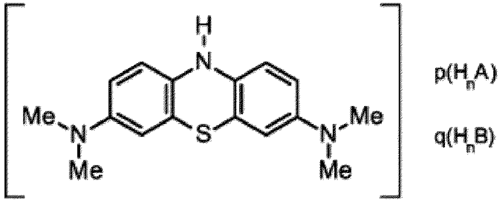
dans laquelle chacun de HₙA et HₙB (lorsqu'ils sont présents) sont des acides protiques qui peuvent être identiques ou différents,
et dans laquelle p = 1 ou 2 ; q = 0 ou 1 ; n = 1 ou 2 ; (p + q) × n = 2,
ou un hydrate ou un solvate de celui-ci.

2. Composé de MT à utiliser selon la revendication 1, dans lequel la dose quotidienne totale est :
(i) supérieure à 35, 40, 50 ou 60 mg et inférieure ou égale à 250 mg de MT par jour pour le sujet ; et/ou
(ii) supérieure ou égale à environ 30,5, 30,6, 31, 35, 37,5, 40, 45, 50, 55, 60, 65, 70, 75, 80, 85, 90, 95, 100, 105, 110, 115, 120, 125, 130, 140, 150, 160, 170, 180, 200, 210, 220, 230, 240 ou 250 mg de MT.

3. Composé à utiliser selon la revendication 1 ou la revendication 2, dans lequel la dose quotidienne totale est d'environ 60, 75 ou 120 mg de MT.

4. Composé à utiliser selon la revendication 1, dans lequel la dose quotidienne totale est d'environ 0,5, 1, 1,5, 2, 2,5, 3, 3,5, 4 mg à environ 5, 6, 7, 8, 9, 10 mg.

5. Composé à utiliser selon l'une quelconque des revendications 1 à 4, dans lequel la dose quotidienne totale du composé est administrée sous forme de dose fractionnée deux fois par jour.

6. Composé à utiliser selon l'une quelconque des revendications 1 à 5 dans lequel le sujet est en outre sélectionné parmi : un sujet hypotenseur dont la PA systolique est inférieure à 80 mmHg ; un sujet en arrêt respiratoire ou cardiaque ; un patient néonatal en détresse ; un sujet avec une suspicion de crise drépanocytaire ; un sujet avec un empoisonnement au monoxyde de carbone.

7. Composé à utiliser selon l'une quelconque des revendications 1 à 6, dans lequel le sujet est diagnostiqué avec une maladie ou une pathologie entraînant une hypoxémie ou en résultant, dans lequel la maladie ou la pathologie est sélectionnée parmi : anémie ; SDRA (syndrome de détresse respiratoire aiguë) ; asbestose ; asthme ; bronchite ; empoisonnement au monoxyde de carbone ; hypoxie cérébrale ; hypoxie cérébrale induite par des forces G excessives (G-LOC) ; malformations cardiaques congénitales chez les enfants ; cardiopathie congénitale chez les adultes ; insuffisance cardiaque congestive ; MPOC(maladie pulmonaire obstructive chronique) ; COVID-19 ; empoisonnement au cyanure ; fibrose kystique ; emphysème ; hypoxie histotoxique ; entraînement à l'hypoventilation ; insomnie ; angio-œdème intermittent ; maladie pulmonaire interstititielle ; hypoxie intra-utérine ; hypoxie ischémique ; lésion pulmonaire, causée par un traumatisme ou une infection, qui est facultativement bactérienne, virale ou fongique ; réponse indésirable à un médicament qui déprime la respiration ; pneumonie ; pneumothorax ; oedème pulmonaire ; embolie pulmonaire ; fibrose pulmonaire ; hypertension pulmonaire ; alcalose respiratoire ; apnée du sommeil ; accident ischémique transitoire ; tuberculose; hypoxie tumorale.

8. Composé à utiliser selon l'une quelconque des revendications 1 à 7, dans lequel le composé contenant du MT est utilisé en association avec une oxygénothérapie supplémentaire.

9. Composé à utiliser selon l'une quelconque des revendications 1 à 8, dans lequel le composé contenant du MT présente la formule suivante, dans laquelle HA et HB sont des acides mono-protiques différents :

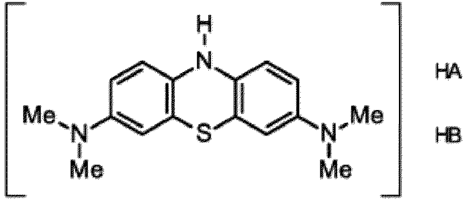

10. Composé à utiliser selon l'une quelconque des revendications 1 à 8, dans lequel le composé contenant du MT présente la formule suivante : 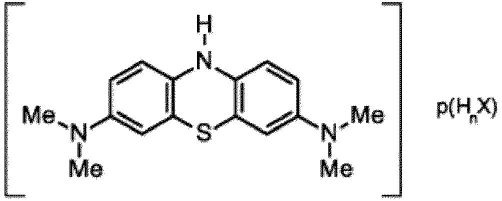 dans laquelle chaque HₙX est un acide protique, dans lequel facultativement le composé contenant du MT présente la formule suivante et est un acide bis-monoprotique :

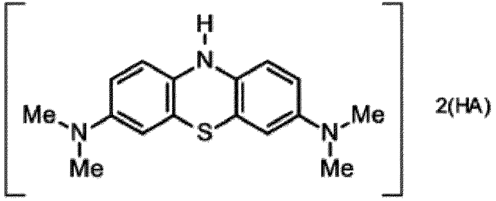

11. Composé à utiliser selon l'une quelconque des revendications 1 à 8, dans lequel le composé contenant du MT présente la formule suivante et H₂A est un acide diprotique :

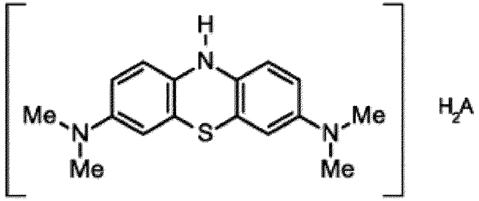

12. Composé à utiliser selon l'une quelconque des revendications 1 à 11 dans lequel le ou chaque acide protique est un acide inorganique, qui est facultativement un acide halogénhydrique ou HNO₃ ou H₂SO₄, dans lequel l'acide halogénhydrique est facultativement choisi parmi HCl ; HBr.

13. Composé à utiliser selon l'une quelconque des revendications 1 à 11, dans lequel le ou chaque acide protique est un acide organique qui est facultativement choisi parmi H₂CO₃ ; CH₃COOH ; de l'acide méthanesulfonique, de l'acide 1,2-éthanedisulfonique, de l'acide éthanesulfonique, de l'acide naphtalènedisulfonique, de l'acide p-toluènesulfonique.

14. Composé à utiliser selon l'une quelconque des revendications 1 à 11, ou selon la revendication 13, dans lequel le composé contant du MT est LMTM :

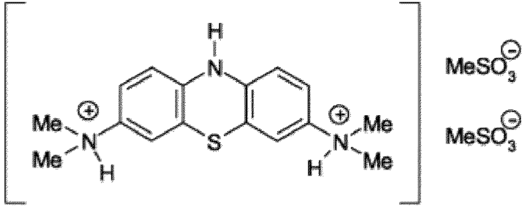

15. Composé à utiliser selon l'une quelconque des revendications 1 à 11, dans lequel le composé contenant du MT est sélectionné dans la liste consistant en :
